# FASCICULE DE BREVET EUROPEEN

(11) **EP 0 636 874 B1**
(45) Date de publication et mention de la délivrance du brevet: **10.01.1996**
(21) Numéro de dépôt: 94420221.7
(22) Date de dépôt: 28.07.1994
(51) Int. Cl.: G01N 1/28, G01F 11/00, C12M 1/26, B01L 11/00

(54) **Procédé de microdosage d'un liquide pour l'obtention des dilutions nanovolumétriques**
Mikrodosierverfahren für Flüssigkeiten zur Erzielung nanovolumetrischer Lösungen
Microdosing method for liquids providing nanovolumetric dilutions

(30) Priorité: 28.07.1993 FR 9309517
(43) Date de publication de la demande: 01.02.1995
(73) Titulaire: BIO MERIEUX, F-69280 Marcy l'Etoile (FR)
(72) Inventeur: Colin, Bruno, F-69160 Tassin La Demi Lune (FR)
(74) Mandataire: Guerre, Dominique

(56) Documents cités:
- EP-A- 0 351 761
- EP-A- 0 508 530
- CH-A- 652 144
- DE-A- 1 598 629
- DE-A- 2 717 963

## Description

La présente invention concerne le dosage, c'est-à-dire le prélèvement puis la libération d'une quantité prédéterminée ou contrôlée d'un liquide ou fluide. Plus précisément, l'invention s'intéresse au micro-dosage d'un liquide, c'est-à-dire au prélèvement puis libération de micro-quantités, par exemple des volumes de l'ordre de quelques microlitres ou de quelques fractions de microlitre d'un liquide.

L'invention trouve notamment application pour l'obtention de dilutions contrôlées, infinitésimales, par exemple nanovolumétriques.

Aujourd'hui, on ne sait pas vraiment doser des micro-quantités d'un liquide, dans des conditions convenables de précision, répétitivité et fiabilité. A cette fin, on utilise généralement des pipettes ou micro-pipettes, par exemple des pipettes commercialisées par la Société GILSON, sous la dénomination commerciale GILSON 90403 P1000, lesquelles prélèvent un volume prédéterminé de liquide, en appliquant une dépression calibrée par la course d'un piston, et libèrent le volume prélevé, en appliquant une surpression au-dessus du piston. Une telle micro-pipette est par exemple décrite dans le document DE-A-1 598 629.

La précision des micro-pipettes est limitée par la tension superficielle du liquide prélevé, les forces capillaires s'exerçant à l'intérieur et à l'extérieur de la paroi de la pipette, et l'accrochage ou rétention de micro-gouttes sur l'extrémité de ladite pipette. La présente invention a pour objet de franchir les limites actuelles des différents dispositifs ou systèmes existants de micro-dosage, en permettant le prélèvement puis la libération de volumes inférieurs au micro-litre, dans de bonnes conditions de précision, fiabilité et répétitivité.

Dans sa forme la plus générale, un procédé de dosage selon l'invention, notamment de micro-dosage, comprend les étapes suivantes :
**(a)** On dispose d'au moins une cavité capillaire, susceptible de contenir le liquide à doser, par exemple fermée ou obturée à une extrémité, dont l'orifice débouchant à l'extérieur sert à la fois à l'introduction et/au vidage dudit liquide, dimensionné de telle sorte que le liquide ne puisse pas entrer ou sortir spontanément de ladite cavité. Avantageusement, la section de l'orifice d'introduction et/ou vidage est choisie inférieure à la section moyenne de la cavité capillaire. Pour un volume interne de la cavité capillaire, au moins égal au volume à doser, la pression interne résiduelle, contrôlée à la fin de l'étape (c) ci-après, détermine l'obtention d'une quantité prédéterminée du liquide, par exemple du liquide à diluer, à la fin de l'étape (d) ci-après.
**(b)** On dispose aussi d'un bain du liquide à doser, par exemple du liquide àdiluer.
**(c)** On vide au moins partiellement la cavité capillaire, au départ exempte de tout liquide, pour amener sa pression interne résiduelle à une valeur inférieure prédéterminée à celle régnant au-dessus du bain liquide lors de l'étape (d), notamment la pression atmosphérique.
**(d)** On met en communication, par l'orifice d'introduction et vidage, l'intérieur de la cavité capillaire partiellement vidée, avec le bain liquide, notamment sous la pression atmosphérique, moyennant quoi on remplit la cavité avec le liquide, par exemple le liquide à diluer.
**(e)** On sépare la cavité capillaire remplie de liquide, ou plus précisément son support l'incorporant, du reste du bain liquide, soit en extrayant ledit support du bain liquide, soit en vidant ou évacuant ledit bain liquide autour dudit support.
**(f)** On vide par tout moyen approprié, et préférentiellement par dépression, le liquide remplissant la cavité capillaire séparée du bain liquide, par exemple dans ou au sein d'un autre bain liquide, à savoir le diluant.

Par "cavité capillaire", on entend tout espace vide à l'intérieur d'un support solide, constitué uniquement d'un ou plusieurs canaux capillaires s'étendant sur une certaine longueur, avec toute conformation appropriée, régulière ou irrégulière, par exemple droite ou en hélice, et dont la section, par exemple circulaire, présente des dimensions (notamment un diamètre) telles que tout liquide s'y introduit ou s'en évacue en formant un ménisque, ou interface liquide/gaz, contenu dans undit canal. Cette cavité capillaire est par nature fermée d'un côté et ouverte de l'autre selon un orifice unique ou multiple, par lequel s'effectue selon la présente invention à la fois l'introduction et le vidage du liquide à doser, notamment une cavité capillaire au sens de la présente invention tout conduit fermé d'un côté et ouvert de l'autre, au sein duquel un liquide est susceptible de s'écouler en régime capillaire, c'est-à-dire, à la manière d'un "piston", sans ménager une interface liquide/gaz dans la direction de la longueur de la cavité capillaire. Préférentiellement, cette cavité présente sensiblement la même section transversale, tant en forme qu'en dimensions, d'une extrémité à l'autre de sa longueur.

Les dimensions effectives de cette cavité capillaire, c'est-à-dire nécessaires pour obtenir en son sein un écoulement capillaire, dépendent de beaucoup de paramètres, tels que viscosité du liquide pénétrant dans la cavité, température de ce dernier, tension superficielle dudit liquide, état et nature de la surface intérieure de la cavité, de telle sorte qu'il est aisé, pour un homme du métier, par des essais de routine, et pour des conditions opératoires maîtrisées, de déterminer les dimensions, telles que diamètre, au-dessous desquelles s'établit un régime d'écoulement capillaire, pour un liquide donné à doser. Préférentiellement, ces essais de routine seront effectués en plaçant l'intérieur de la cavité en communication par l'orifice d'introduction/vidage avec un bain du liquide à doser, ceci en relation sensiblement isobare entre l'intérieur gazeux de la cavité et le bain du liquide, et pour différentes dimensions transversales (ou diamètres) de la cavité.

Quant au support solide, il peut s'agir soit d'un support libre, tel qu'une bille, soit d'un support fixe, par exemple monté sur la paroi du contenant pour la réception du bain liquide.

Grâce à la présente invention, si le volume de la cavité capillaire est au moins égal au volume à doser, la quantité remplissant ladite cavité lors de l'étape (d) est fonction et peut être contrôlée par la seule pression interne résiduelle à la fin de l'étape (c). Le volume ainsi prélevé peut être bien inférieur du volume du capillaire, tout en restant maîtrisable.

Et cette quantité peut être prélevée et restituée sans micro-gouttes résiduelles, du côté de l'orifice d'introduction/vidage.

Comme déjà dit, le procédé selon l'invention peut être mis en oeuvre pour l'obtention de dilutions contrôlées, notamment successives ou "en cascade", à partir de l'échantillon prélevé et restitué de manière dosée par la cavité capillaire. Grâce aux micro-échantillons obtenus selon l'invention, on peut obtenir de manière précise des dilutions infinitésimales, et ceci, sans manipuler de grandes quantités de liquide ou diluant, ce qui constitue un avantage décisif de la présente invention.

Le procédé selon l'invention peut être utilisé aussi pour le transfert d'une quantité dosée d'un liquide, notamment d'une micro-quantité, vers un récipient, ou un support tel que lame, film, puits, cuvette, ou analogue.

Le procédé selon l'invention peut être utilisé pour ajouter une micro-quantité dosée d'un liquide, à une quantité plus importante, également dosée, du même liquide.

Enfin, le procédé selon l'invention peut être mis en oeuvre de manière automatique, avec un automate approprié.

La présente invention est maintenant décrite par référence au dessin annexé, dans lequel :
- les figures 1 à 5 représentent, de manière schématique et dans sa forme générale, les différentes étapes d'un procédé de dosage selon l'invention ;
- les figures 6 à 8 représentent, de manière schématique et par voie automatique, un premier mode d'exécution d'un procédé de micro-dosage selon l'invention, par exemple dans le cadre d'un appareil automatique ou automate de bactériologie ;
- les figures 9 à 13 représentent, toujours de manière schématique, et par voie automatique, un deuxième mode d'exécution d'un procédé de micro-dosage selon l'invention, mis en oeuvre pour effectuer une dilution contrôlée, toujours dans le cadre d'un automate de bactériologie ;
- la figure 14 représente, de manière schématique, un dispositif expérimental ayant permis de tester et quantifier les principes selon l'invention.

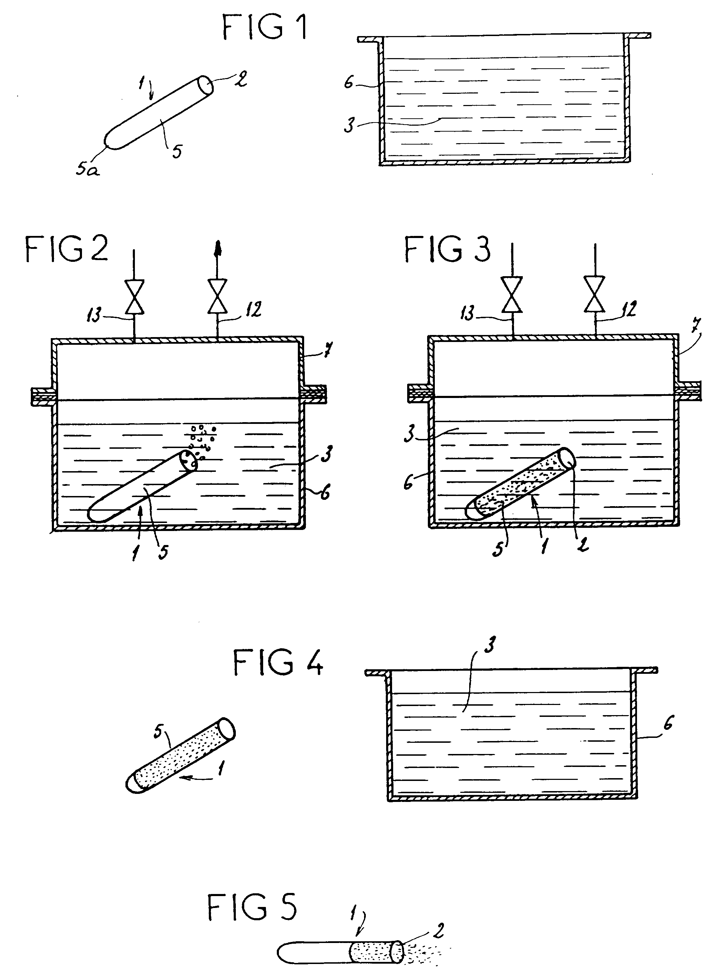

Selon les figures 1 à 5, les dimensions de la cavité ou tube capillaire 1 ont été volontairement agrandies, de manière à rendre plus explicites les développements ci-après.

De manière générale, un dispositif de dosage selon l'invention, permettant de prélever, puis restituer une quantité prédéterminée d'un liquide comprend:
- un moyen ou support 5 de dosage, représenté à la figure 1 sous la forme d'un tube capillaire avec un fond ou extrémité 5a fermée, servant au prélèvement puis à la libération du liquide. Ce support 5 détermine ou comprend une cavité capillaire 1, susceptible de contenir ledit liquide, débouchant à l'extérieur par un orifice 2 d'introduction, puis vidage du même liquide, dimensionné de telle sorte que le liquide ne puisse pas entrer ou sortir spontanément de ladite cavité. L'orifice 2 a préférentiellement une dimension transversale, telle que diamètre, plus faible que la dimension transversale moyenne de la cavité capillaire 1 selon sa longueur
- un contenant ou cellule 6 susceptible de contenir un bain 3 du liquide à doser, et de recevoir le support 5, dont l'intérieur de la cavité capillaire 1 est susceptible de ce fait, de communiquer avec l'intérieur du contenant 6, par l'orifice 2. Un couvercle ou bouchon 7 est susceptible d'obturer, de manière étanche, le contenant 6, de façon à ménager au-dessus du bain liquide 3 un ciel gazeux, dont la pression peut être contrôlée; ce couvercle ou bouchon 7 est équipé d'un moyen 12 de mise en dépression et/ou mise sous pression, par exemple à l'air libre, et d'un moyen 13 d'introduction et/ou d'évacuation du liquide, dont l'extrémité basse peut être prolongée jusqu'au niveau du fond du contenant 6, de manière à pouvoir vider le contenant jusqu'à ce niveau inférieur.

Conformément à la figure 1, on dispose donc au départ de la cavité capillaire 1, fermée à une extrémité 5a et ouverte à une extrémité 2, et du bain 3 du liquide à doser, par exemple d'un liquide à diluer.

Conformément à la figure 2, on introduit le support capillaire 5, et par conséquent la cavité capillaire 1 au sein du bain liquide 3. On dispose sur le contenant 6 le couvercle ou bouchon 7, et par le moyen 12, relié à une source de vide non représentée, on vide la cavité 1, au départ exempte de tout liquide, pour amener sa pression interne à une valeur résiduelle inférieure à celle régnant au-dessus du bain liquide 3 lors de l'étape suivante décrite par référence à la figure 3, à savoir la pression atmosphérique.

Conformément à la figure 3, toujours par le moyen 12, on rétablit la pression atmosphérique à l'intérieur de l'enceinte définie par le contenant 6 et le couvercle 7, moyennant quoi on remplit la cavité capillaire 1, en mettant en communication l'intérieur de cette dernière, par l'orifice 2, avec le bain liquide 3. La progression du liquide à l'intérieur du tube s'effectue de manière capillaire, avec un ménisque se déplaçant vers le fond 5a de la cavité capillaire 5. La position finale de ce ménisque dépend de la pression résiduelle obtenue à la fin de l'étape selon figure 3. En définitive, le volume prélevé par la cavité capillaire 1 dépend, pour un volume de ladite cavité supérieur au volume à prélever, de sa pression interne résiduelle avant remplissage.

Par référence à la figure 4, on sépare la cavité 1 remplie du reste du bain liquide 3. Comme indiqué précédemment, cette séparation pourrait être obtenue en vidant l'intérieur du contenant 6, par le moyen 13 prolongé jusqu'au fond du contenant 6.

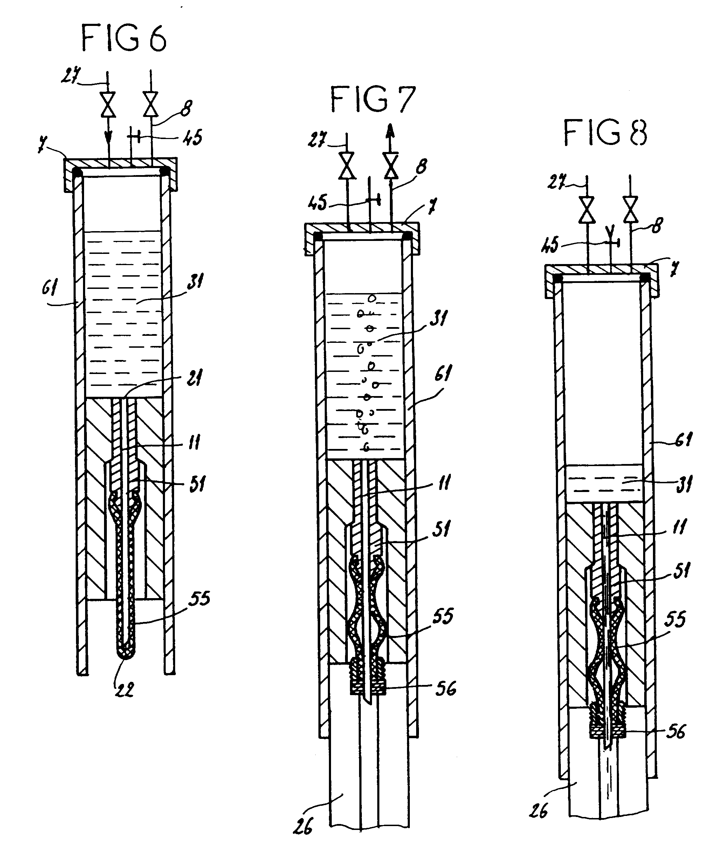

Conformément à la figure 5, on vide la cavité remplie 1, séparée du bain liquide 3, par tout mode approprié, par exemple :
- l'application d'une force centrifuge passant par l'orifice 2 ;
- l'application à l'extérieur de la cavité 1, d'une pression externe inférieure à la pression interne dans ladite cavité ;
- l'application à l'intérieur de la cavité 1, d'une pression supérieure à sa pression interne ;
- l'application d'une poussée sur le volume liquide remplissant la cavité 1, vers l'extérieur de cette dernière.

La progression du liquide à l'extérieur du tube 1 s'effectue également de manière capillaire, avec un ménisque se déplaçant vers l'orifice 2 de la cavité capillaire 5, et sortant de ce dernier.

Conformément à la figure 2, le vidage de la cavité capillaire 1, préalable à son remplissage avec le liquide, est effectué, en disposant cette cavité au contact du bain liquide 3, par son orifice 2, et en appliquant au-dessus du bain 3, une pression inférieure à celle régnant au-dessus du bain 3, lors de l'étape suivante décrite par référence à la figure 3, à savoir la pression atmosphérique. Bien entendu, ce vidage pourrait être obtenu en dehors du bain liquide, par exemple en disposant le support 5 au sein de l'enceinte définie par la réunion étanche du contenant 6 et du couvercle 7, en vidant l'air de ladite enceinte, puis en y introduisant le bain liquide 3.

Il résulte de la description précédente que :
- le moyen de vidage de la cavité capillaire 1 du moyen de dosage 5, au départ exempte de tout liquide, par son orifice 2, est le moyen 12 de mise en dépression ;
- le moyen de séparation de la cavité capillaire 1 remplie de liquide, du reste du bain liquide 3, peut être le moyen 13 d'extraction du liquide, servant notamment de moyen de pompage de cette dernière.

Pour procéder à une dilution contrôlée, en particulier à une micro-dilution, après l'étape selon la figure 4, on dispose la cavité capillaire 1 remplie du liquide à diluer, au contact d'un autre bain liquide, à savoir du diluant, ceci par l'orifice 2, et on vide cette cavité 1 remplie, au sein ou dans l'autre bain liquide, à savoir une quantité déterminée, de préférence dosée du diluant, par exemple en appliquant, comme décrit ci-après, au-dessus d'un bain de ce dernier, une pression inférieure à la pression interne de la cavité 1.

Le même contenant ou récipient 6, équipé de son couvercle ou bouchon 7, peut servir à la réception, et du liquide à diluer et du diluant liquide.

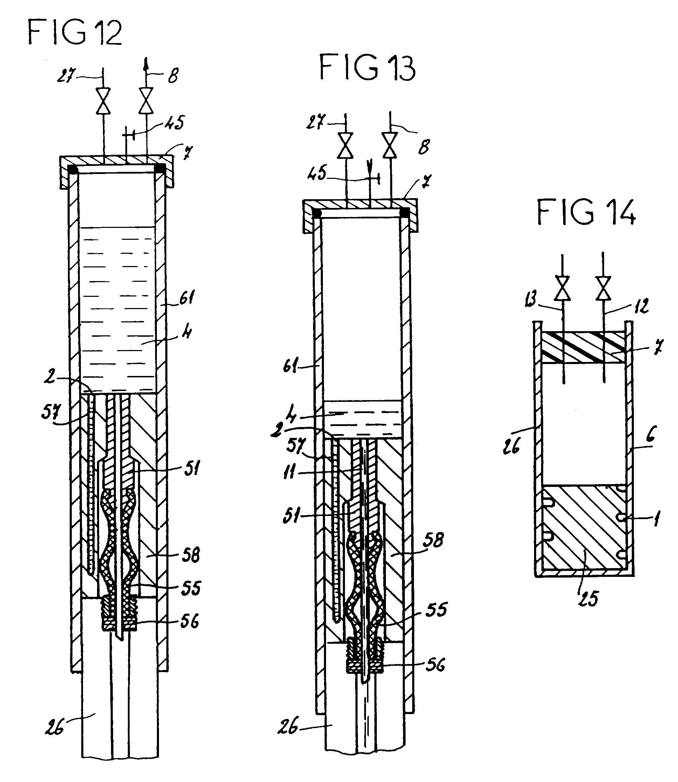

L'efficacité du principe de dosage et éventuellement dilution décrit précédemment a été testée au moyen du dispositif représenté à la figure 14, permettant un dosage par spectrophotométrie. Sur cette figure, les références numériques identiques à celles déjà utilisées par référence aux figures 1 à 5, désignent les mêmes éléments ou composants fonctionnels. Selon la figure 14, la cavité capillaire 1 est obtenue en ayant ménagé, par tout moyen approprié, par exemple par usinage, une rainure hélicoïdale sur la surface extérieure d'un noyau cylindrique 25, lequel est disposé de manière étanche au fond de la paroi cylindrique d'un tube 26; la rainure hélicoïdale débouche dans le contenant 6, déterminé par le volume libre dans le tube 26, et est fermée à son extrémité opposée.

A titre d'exemple, le contenant 6 représente un volume de 7 ml, et la cavité capillaire 1, en forme d'hélice, un volume de 55 µl. Les parois du tube 26 sont transparentes, de manière à permettre des mesures optiques, et en particulier, des mesures de densité optique.

Avec ce dispositif expérimental, le mode opératoire suivant est mis en oeuvre :
- au départ, la cavité capillaire hélicoïdale 1 est vide de liquide, et en particulier, à pression atmosphérique; son remplissage est impossible par simple capillarité au contact du liquide à doser
- le contenant 6 reçoit une solution constituée par de l'encre ou un colorant dilué dans de l'eau déminéralisée, dans la proportion d'un volume d'encre pour cent volumes d'eau ; cette solution constitue le bain liquide de départ
- par le moyen 12, pouvant consister en une simple seringue traversant le bouchon 7, le contenant 6 est mis en dépression, par aspiration d'un volume d'air prédéterminé ; ceci vide l'air de la cavité capillaire 1, au sens où cette dernière est également mise en dépression jusqu'à une pression interne résiduelle contrôlée
- toujours par le moyen 12, l'intérieur de la cavité 6 revient à la pression atmosphérique, moyennant quoi, la cavité capillaire 1 se remplit avec la solution
- l'excédent de solution est extrait du contenant 6, par exemple par pompage ou simple vidage, et l'intérieur du contenant 6 est lavé avec de l'eau déminéralisée, à la pression atmosphérique, de manière à éviter toute entrée du liquide de lavage dans la cavité capillaire 1, et/ou sortie du liquide de la cavité
- 2 ml ou tout autre volume d'un diluant, par exemple de l'eau déminéralisée, sont ensuite introduits dans le contenant 6, pour constituer l'autre bain liquide au sens de la définition générale précédente
- le contenant 6 est ensuite mis sous dépression, par le même moyen 12, moyennant quoi, la solution d'encre initiale, piégée par la cavité capillaire 1, est extraite dans le contenant 6, pour obtenir un mélange du diluant et de cette solution initiale
- 1 ml de ce mélange est prélevé et dosé sur un spectrophotomètre "Reaction Rate Photometer" commercialisé par la Société VITATRON, pour une lecture de densité optique à 620 nm
- le cycle décrit précédemment peut être répété plusieurs fois, le mélange obtenu à la fin du cycle précédent constituant le bain liquide de départ du cycle suivant.

Par étalonnage du spectophotomètre, on peut mettre en correspondance la densité optique de chaque dilution ou sous-dilution, et la concentration en encre de cette dernière. Pour une dilution donnée, on procède à cinq manipulations différentes, pour obtenir une valeur moyenne.

On obtient, pour l'ensemble de ces expériences, le tableau N° 1 ci-après, dans lequel :
- la première colonne exprime, en %, la concentration théorique en solution primaire ou de départ du mélange dosé optiquement, obtenue à partir du nombre de dilutions et/ou du volume de diluant utilisé
- la deuxième colonne exprime la densité optique du mélange seul, exprimée en mDO
- la troisième colonne exprime l'écart type
- la quatrième colonne exprime le coefficient de variation

**Tableau N° 1**

| conc (%) | mDO | ET | CV |
|---|---|---|---|
| 0,1 | 6,6 | 0,5 | 8,2% |
| 0,2 | 11,8 | 1,1 | 9,3% |
| 0,4 | 20,8 | 2,1 | 10% |
| 0,6 | 32,2 | 3,2 | 10% |
| 0,8 | 40,8 | 1,5 | 3,6% |
| 0,9 | 45,6 | 0,5 | 1,2% |
| 0,95 | 47,5 | 1,2 | 2,6% |
| 1 | 50,6 | 2,4 | 4,7% |
| 1,5 | 73,4 | 1,5 | 2% |
| 1,75 | 86 | 1,4 | 1,6% |
| 2 | 92 | 1 | 1% |
| 2,25 | 108 | 1,6 | 1,5% |
| 2,5 | 120,4 | 2,5 | 2,1% |
| 3 | 139,4 | 2,3 | 1,6% |

### Densité optique en fonction de la concentration en solution primaire

En corrélant ces valeurs, en portant les concentrations en abscisse et les densités optiques en ordonnée, on constate une corrélation quasi linéaire entre la concentration et la densité optique, selon la loi suivante :
mDO = concentration (en %) x 46 + 3,5
avec un coefficient de corrélation linéaire r = 0,9992. On a ensuite recherché quelle était l'influence de la pression interne résiduelle, à la fin du vidage de la cavité capillaire, sur la précision ou valeur du volume liquide prélevé puis restitué par cette cavité.

Conformément au tableau N°2, on part d'un bain liquide ou solution primaire d'encre dans l'eau, dont le volume est donné en ml comme indiqué dans la première colonne. Chaque manipulation est effectuée cinq fois à une pression déterminée, et les valeurs exprimées ci-après correspondent à la moyenne.

**Tableau N° 2**

| ml | mDO | ET | CV | dilution(%) |
|---|---|---|---|---|
| 1 | 20 | 7 | 36% | 0,35 |
| 2 | 38 | 3 | 7% | 0,75 |
| 3 | 51 | 6 | 12% | 1,03 |
| 4 | 58 | 3 | 6% | 1,18 |
| 5 | 70 | 3 | 4% | 1,45 |

### Densité optique et dilution en fonction de la dépression

Conformément au tableau N°3, on effectue les mêmes manipulations que celles définies par référence au tableau N°2, mais en utilisant une source de vide contrôlée par un manomètre, dont la valeur de réglage, exprimée en millibars, est donnée dans la première colonne du tableau N° 3 ci-après. [1 mBar = 10 N/m]

**Tableau N°3**

| mBar | mDO | ET | CV | dilution (%) |
|---|---|---|---|---|
| 50 | 4,6 | 1,8 | 39% | 0,024 |
| 75 | 8,8 | 0,8 | 9% | 0,115 |
| 100 | 11,2 | 1,3 | 12% | 0,167 |
| 150 | 19,4 | 1,14 | 6% | 0,346 |
| 200 | 28 | 2,1 | 8% | 0,532 |
| 270 | 37,2 | 1,8 | 5% | 0,733 |
| 300 | 43,4 | 1,8 | 4% | 0,867 |
| 400 | 57,2 | 2,7 | 5% | 1,167 |
| 500 | 70,8 | 1,6 | 2% | 1,463 |
| 600 | 82,6 | 2,4 | 3% | 1,719 |
| 700 | 95,8 | 1,9 | 2% | 2,007 |
| 800 | 120,2 | 1,6 | 1% | 2,537 |

### Densité optique et dilution en fonction de la dépression (les valeurs ont été calculées à partir de la corrélation linéaire ci-dessus)

En conclusion de ces tableaux N° 2 et 3, on constate que, lorsque la pression interne résiduelle avant remplissage est inférieure à 400 millibars, la dilution demeure proportionnelle à la pression résiduelle. Au-delà de cette valeur, la dilution tend vers une limite qui correspond à l'approche du vide total dans la cavité capillaire.

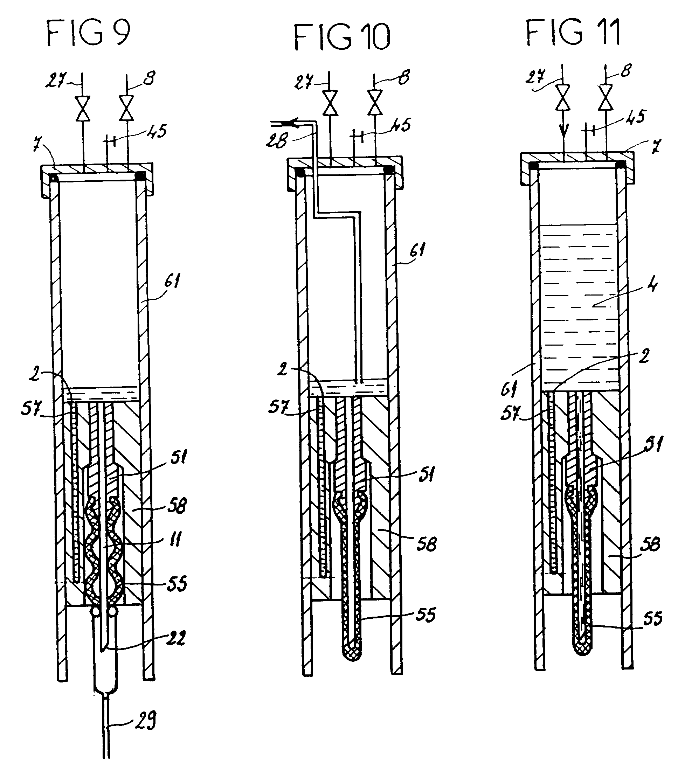

Conformément aux figures 6 à 8, on décrit, de manière schématique, un premier mode de dosage d'un liquide selon l'invention, au moyen d'un tube 61, se terminant par une aiguille perforatrice 51, obturée par un septum 55, déterminant un conduit capillaire droit 11, lequel comporte deux orifices 21 et 22, le premier 21 du côté du tube 61, et le second 22 du côté de la sortie de l'aiguille 51 ; il est entendu que le diamètre de l'orifice 22 peut varier, dans la mesure où il permet de conserver un régime capillaire. Un tel tube est destiné à coopérer avec un récipient creux ou cassette ou carte 26, obturée par une valve 56 d'introduction, comportant un opercule perforable visco-élastique. Un bouchon 7 ferme la partie supérieure du tube 61, et est équipé, d'un moyen 8 en relation avec une source de vide, d'un moyen 27 en relation avec une alimentation en solution à doser et diluer, un moyen 28 de pompage (représenté uniquement à la figure 10) à l'intérieur du tube 61, et un moyen 45 de mise à l'air de l'intérieur du tube 61.

Dans un automate de mesure biologique, notamment bactériologique, selon le premier mode d'exécution de l'invention, les étapes suivantes sont mises en oeuvre; pour remplir successivement une cassette avec le liquide à diluer, et une autre cassette avec le même liquide dilué dans un diluant
- conformément à la figure 6, on remplit le tube 61 avec une quantité donnée d'un liquide 31 à diluer, en ml ;
- conformément à la figure 7, l'aiguille 51 pénètre dans la valve 56 de manière étanche, on place sous vide au moins partiel l'intérieur du tube rempli 61, de manière, d'une part à vider l'air contenu dans la cavité capillaire 11 de l'aiguille perforatrice 51, et d'autre part à vider au moins partiellement l'air de la cassette 26 ;
- conformément à la figure 8, et toujours avec l'aiguille 51 pénétrant dans la valve 56, en rétablissant la pression atmosphérique à l'intérieur du tube 61, on remplit la cassette 26 avec un volume prédéterminé du liquide à diluer ;
- le remplissage de la cassette 26 étant achevé, le tube 61 est retiré par rapport à la cassette 26, l'aiguille perforatrice 51 se trouve à nouveau obturée à une extrémité par le septum 55, et la cavité capillaire 11 piège ainsi une quantité déterminée de liquide à diluer;
- le volume résiduel 31 dans le tube 61 est évacué, par exemple au moyen du conduit 28 représenté à la figure 10, et relié à une source de vide ;
- le tube 61 est rempli par une quantité déterminée de diluant, comme déjà décrit par référence à la figure 6 ;
- comme déjà décrit par référence à la figure 7, l'aiguille 51 pénètre dans la valve 56 d'une nouvelle cassette, et on place sous vide au moins partiel l'intérieur du tube 61 rempli de diluant, pour vider au moins partiellement l'air contenu dans la nouvelle cassette, et vider le liquide à diluer contenu dans la cavité capillaire 11, en effectuant ainsi une dilution de ce dernier ;
- comme déjà décrit par référence à la figure 8, on remplit la nouvelle cassette avec la dilution obtenue.

Conformément aux figures 9 à 13, on met en oeuvre un deuxième mode d'exécution de l'invention, pour lequel les moyens précédemment décrits par référence aux figures 6 à 8 sont modifiés, pour prévoir une cavité capillaire 57 droite et borgne au sein du noyau 58 obturant le tube 61, communiquant avec la partie supérieure de ce dernier.

On part de l'état représenté à la figure 9 ou 10, selon lequel la cavité 57 a été remplie avec le liquide à diluer, et une quantité résiduelle du même liquide est présente dans le tube 61. Cet état peut être obtenu en effectuant les étapes décrites précédemment par référence aux figures 6 à 8.

A partir de cet état, les étapes suivantes sont effectuées :
- conformément à la figure 9, on vide, par son extrémité aval 22 l'aiguille perforatrice 51, avec perforation du septum 55, grâce à une source de vide 29 appliquée de manière étanche à l'extrémité basse de l'aiguille 51, jusqu'à obtenir le vidage complet du tube 61 et de l'aiguille 51, la cavité 57 restant pleine ;
- ou selon la figure 10, sans perforation du septum 55, on pompe le liquide à l'intérieur du tube 61, jusqu'à obtenir le vidage du tube 61, la cavité 57 restant pleine ; bien entendu, dans ce mode de réalisation, il est impossible de vider l'aiguille 51 ; aussi, pour ne pas perturber de façon trop importante la précision de la dilution, il est impératif que le volume de l'aiguille 51 soit pratiquement négligeable par rapport au volume de la cavité 57; ceci peut être obtenu avec une cavité capillaire hélicoïdale selon le mode d'exécution décrit à la figure 14; et le vidage du tube 61 doit s'effectuer sous pression atmosphérique pour ne pas créer une dépression, même partielle et/ou temporaire, susceptible de vider même partiellement la cavité 57, par le conduit 28 relié à une source de vide non représentée ;
- conformément à la figure 11, on remplit ensuite l'intérieur du tube 61, par le moyen 27, avec un diluant
- conformément à la figure 12, par communication entre l'aiguille 51 et la valve 56, on met sous dépression, l'intérieur du tube 61 et de la cassette 26, moyennant quoi la cavité capillaire 57 restitue au sein du diluant la quantité du liquide à diluer précédemment piégée dans cette même cavité; il y a donc mélange et dilution ;
- conformément à la figure 13, en mettant à l'air libre l'intérieur du tube 61, et en établissant une liaison entre l'aiguille 51 et l'intérieur de la cassette 26, on vide dans la cassette le volume dilué ainsi obtenu.

## Revendications

1. Procédé de dosage d'une quantité prédéterminée d'un liquide, **caractérisé en ce que** :
**(a)** on dispose d'au moins une cavité capillaire (1,11,57) susceptible de contenir ledit liquide, dont l'orifice (2,21) débouchant à l'extérieur sert à la fois à l'introduction et/au vidage dudit liquide, et est dimensionné de telle sorte que le liquide ne puisse pas entrer ou sortir spontanément de ladite cavité, notamment de section inférieure à celle de la cavité capillaire ;
**(b)** on dispose d'un bain (3, 31) dudit liquide ;
**(c)** on vide au moins partiellement ladite cavité (1,11,57), au départ exempte de tout liquide, pour amener sa pression interne résiduelle à une valeur inférieure à celle régnant au-dessus du bain liquide (3,31) lors de l'étape (d), notamment la pression atmosphérique ;
**(d)** on met en communication, par l'orifice (2,21), l'intérieur de la cavité capillaire (1,11,57) au moins partiellement vidée, avec le bain liquide (3,31), notamment sous la pression atmosphérique, moyennant quoi on remplit ladite cavité ;
**(e)** on sépare la cavité capillaire (1,11,57) remplie, du reste du bain liquide (3,31) ;
**(f)** on vide la cavité capillaire remplie (1,11,57), séparée du bain liquide (3,31).

2. Procédé selon la revendication 1, caractérisé en ce qu'on effectue l'étape (c), en disposant ladite cavité (1,11,57) dans le bain liquide (3,31), et en appliquant au-dessus de ce dernier une pression inférieure à celle régnant au-dessus du bain liquide (3, 31) lors de l'étape (d).

3. Procédé selon la revendication 1, caractérisé en ce qu'on effectue l'étape (f) selon l'un quelconque des modes définis ci-après, à savoir :
**3.1)** l'application d'une force centrifuge passant par l'orifice d'introduction (2) ;
**3.2)** l'application à l'extérieur de la cavité (1, 11) d'une pression externe inférieure à sa pression interne ;
**3.3)** l'application à l'intérieur de la cavité (1, 11) d'une pression supérieure à sa pression interne;
**3.4)** le volume liquide remplissant la cavité (1, 11) est poussé à l'extérieur de cette dernière.

4. Procédé selon la revendication 3, selon lequel on effectue l'étape (f) par le mode 3.2, caractérisé en ce qu'on dispose la cavité (1,11,57), dans un autre bain liquide (4), et on applique au-dessus dudit bain une pression inférieure à la pression interne de la cavité (1,11,57).

5. Méthode de dilution contrôlée d'un liquide, mettant en oeuvre un procédé de dosage selon l'une quelconque des revendications 1 à 4, caractérisée en ce que, d'une part le liquide à diluer (1,11,57) constitue le bain liquide selon les étapes (a) à (e), et d'autre part, selon l'étape (f), on vide la cavité (31) remplie dans une quantité dosée d'un diluant liquide (4).

6. Méthode de dilution selon les revendications 1, 4 et 5, caractérisée en ce que la quantité dosée du diluant liquide (4) constitue ledit autre bain liquide selon l'étape (f) du procédé de dosage.

7. Dispositif de dosage d'une quantité prédéterminée d'un liquide, caractérisé en ce qu'il comprend :
**(A)** un moyen ou support (5,51) de dosage, servant au prélèvement puis à la libération du liquide, comprenant une cavité (1,11,57) capillaire, susceptible de contenir ledit liquide, dont l'orifice (2,21) débouchant à l'extérieur sert à la fois à l'introduction et/au vidage dudit liquide, dimensionné de telle sorte que le liquide ne puisse pas entrer ou sortir spontanément de ladite cavité, notamment de section inférieure à celle de la cavité capillaire ;
**(B)** un contenant (6,61) susceptible de contenir un bain (3,31) dudit liquide, dont l'intérieur est susceptible de communiquer avec l'intérieur de la cavité (1,11,57) du moyen de dosage, par l'orifice (2,21) de la cavité ;
**(C)** un moyen de vidage (8,12) de la cavité (1,11,57) du moyen de dosage, au départ exempte de tout liquide, par son orifice (2,21) ;
**(D)** un moyen de séparation de la cavité remplie du moyen de dosage, du reste du bain liquide (3,31) ;
**(E)** un moyen de vidage de la cavité remplie de liquide, mais séparée du reste du liquide.

8. Dispositif de dosage selon la revendication 7, caractérisé en ce que le moyen de vidage consiste en un moyen (12) de mise en dépression du ciel gazeux au-dessus du contenant.

9. Dispositif selon la revendication 7, caractérisé en ce que le moyen de séparation de la cavité remplie, du reste du bain liquide, est, ou un moyen de pompage de cette dernière, ou un moyen d'aspiration de cette dernière par un orifice calibré de la cavité.

10. Dispositif selon la revendication 7, caractérisé en ce que le moyen de vidage de la cavité remplie de liquide est un moyen d'application à ladite cavité d'une pression inférieure à sa pression interne.

11. Dispositif de dosage selon l'une quelconque des revendications 7 à 11, caractérisé en ce que l'orifice d'introduction/vidage (2,21) à une dimension transversale, telle que diamètre, plus faible que la dimension transversale moyenne de la cavité capillaire, selon sa longueur.

12. Dispositif de dilution contrôlée d'un liquide, incorporant un dispositif de dosage selon l'une quelconque des revendications 7 à 10, caractérisé en ce que le même contenant (6, 61) sert à la réception, et du liquide à diluer et du diluant liquide.

13. Dispositif de dilution selon la revendication 7, caractérisé en ce que le moyen ou support de dosage (5) est une aiguille (51) déterminant le conduit capillaire (11) et comportant deux orifices (21,22), l'un communiquant avec le contenant (61), et l'autre avec l'extérieur ou un récipient.

14. Dispositif selon la revendication 12, caractérisé en ce que le contenant (61) est en relation avec un moyen (12) de mise en dépression et/ou mise sous pression, et un moyen (13) d'introduction et/ou d'évacuation du liquide à diluer.

## Patentansprüche

1. Verfahren zum Dosieren einer bestimmten Menge einer Flüssigkeit, dadurch gekennzeichnet,
(a) daß zumindest ein kapillarer Hohlraum (1, 11, 57) vorgesehen wird, der die Flüssigkeit aufnehmen kann, dessen Öffnung (2, 21), die nach außen mündet, zugleich zum Einfüllen und zum Entleeren der Flüssigkeit dient, und die so dimensioniert ist, daß die Flüssigkeit nicht von selbst in den Hohlraum eintritt oder aus diesem austritt, wobei diese insbesondere einen Querschnitt aufweist, der kleiner als der des kapillaren Hohlraumes ist;
(b) daß ein Bad (3, 31) an dieser Flüssigkeit bereitgestellt wird;
(c) daß der Hohlraum (1, 11, 57), der anfänglich frei von jeder Flüssigkeit ist, zumindest teilweise evakuiert wird, so daß dessen verbleibender Innendruck auf einen Wert eingestellt wird, der unter dem Wert liegt, der in Schritt (d) über dem Flüssigkeitsbad (3, 31) herrscht, insbesondere der Atmosphärendruck;
(d) daß man über die Öffnung (2, 21) den zumindest teilweise evakuierten Innenraum des kapillaren Hohlraumes (1, 11, 57) mit dem Flüssigkeitsbad (3, 31), insbesondere unter Atmosphärendruck stehend, in Verbindung bringt, wodurch der Hohlraum gefüllt wird;
(e) daß der gefüllte kapillare Hohlraum (1, 11, 57) vom Rest des Flüssigkeitsbades (3, 31) getrennt wird; und
(f) daß der gefüllte kapillare Hohlraum (1, 11, 57), getrennt vom Flüssigkeitsbad (3, 31), entleert wird.

2. Verfahren nach Anspruch 1, dadurch gekennzeichnet, daß der Schritt (c) derart durchgeführt wird, daß der Hohlraum (1, 11, 57) im Flüssigkeitsbad (3, 31) vorgesehen ist, und daß über letzterem ein Druck angelegt wird, der unter dem liegt, der während des Schrittes (d) über dem Flüssigkeitsbad (3, 31) herrscht.

3. Verfahren nach Anspruch 1, dadurch gekennzeichnet, daß der Schritt (f) nach einer der nachfolgend definierten Art und Weisen durchgeführt wird, nämlich
3.1) Anwenden einer Zentrifugalkraft, die durch die Einführöffnung (2) hindurch gerichtet ist;
3.2) Anlegen eines äußeren Druckes an der Außenseite des Hohlraumes (1, 11) der geringer ist als dessen Innendruck;
3.3) Anlegen eines Druckes im Inneren des Hohlraumes (1, 11), der über dessen Innendruck liegt;
3.4) Herausdrücken des Flüssigkeitsvolumens, das den Hohlraum (1, 11) füllt, aus letzterem.

4. Verfahren nach Anspruch 3, bei dem im Schritt (f) die Variante 3.2 eingesetzt wird, dadurch gekennzeichnet, daß der Hohlraum (1, 11, 57) in ein anderes Flüssigkeitsbad (4) eingebracht wird, und daß über diesem Bad ein Druck angelegt wird, der unter dem Innendruck des Hohlraumes (1, 11, 57) liegt.

5. Verfahren zum gesteuerten Verdünnen einer Flüssigkeit, bei dem ein Dosierverfahren nach einem der Ansprüche 1 - 4 eingesetzt wird, dadurch gekennzeichnet, daß zum einen die zu verdünnende Flüssigkeit (1, 11, 57) das Flüssigkeitsbad der Schritte (a) bis (e) darstellt, und daß zum anderen beim Schritt (f) der gefüllte Hohlraum (31) in eine bestimmte Menge des flüssigen Verdünnungsmittels (4) entleert wird.

6. Verfahren zum Verdünnen nach den Ansprüchen 1, 4 und 5, dadurch gekennzeichnet, daß die bestimmte Menge des flüssigen Verdünnungsmittels (4) beim Schritt (f) des Dosierverfahrens das andere Flüssigkeitsbad bildet.

7. Vorrichtung zum Dosieren einer bestimmten Menge einer Flüssigkeit, dadurch gekennzeichnet, daß sie folgendes aufweist, nämlich
(A) ein Dosismittel oder einen Dosisträger (5, 51), der zum Entnehmen und anschließend zum Freigeben der Flüssigkeit dient, und der einen kapillaren Hohlraum (1, 11, 57) aufweist, der die Flüssigkeit aufnehmen kann, wobei dessen Öffnung (2, 21), die nach außen mündet und die zugleich zum Einfüllen und zum Entleeren der Flüssigkeit dient, so dimensioniert ist, daß die Flüssigkeit nicht von selbst in den Hohlraum eintritt oder aus diesem austritt, wobei diese insbesondere einen Querschnitt aufweist, der kleiner als der des kapillaren Hohlraumes ist;
(B) ein Behältnis (6, 61), das ein Bad (3, 31) der Flüssigkeit aufnehmen kann, wobei der Innenraum des Behältnisses mit dem Inneren des Hohlraumes (1, 11, 57) des Dosismittels über die Öffnung (2, 21) des Hohlraumes (2, 21) kommuniziert;
(C) Mittel (8, 12) zum Evakuieren des anfänglich frei von jeglicher Flüssigkeit befindlichen Hohlraums (1, 11, 57) der Dosismittel über dessen Öffnung (2, 21);
(D) Mittel zum Trennen des gefüllten Hohlraumes der Dosismittel vom Rest des Flüssigkeitsbades (3, 31);
(E) Mittel zum Entleeren des mit Flüssigkeit gefüllten Hohlraumes, jedoch getrennt vom Rest der Flüssigkeit.

8. Vorrichtung zum Dosieren nach Anspruch 7, dadurch gekennzeichnet, daß die Mittel zum Entleeren ein Mittel (12) darstellen, die den Gasraum über dem Behältnis unter Unterdruck setzen.

9. Vorrichtung nach Anspruch 7, dadurch gekennzeichnet, daß die Mittel zum Trennen des gefüllten Hohlraumes vom Rest des Flüssigkeitsbades entweder Mittel zum Abpumpen des letzteren oder Mittel zum Absaugen des letzteren über eine Kalibrieröffnung des Hohlraumes darstellen.

10. Vorrichtung nach Anspruch 7, dadurch gekennzeichnet, daß die Mittel zum Entleeren des mit Flüssigkeit gefüllten Hohlraumes ein Mittel zum Anlegen eines Druckes in dem Hohlraum unter dessen Innendruck ist.

11. Vorrichtung zum Dosieren nach einem der Ansprüche 7 - 11, dadurch gekennzeichnet, daß die Einführ-/Entleeröffnung (2, 21) in Querrichtung ein Maß, wie beispielsweise den Durchmesser aufweist, das geringer als das mittlere Quermaß des kapillaren Hohlraumes, über dessen Länge gesehen, ist.

12. Vorrichtung zum gesteuerten Verdünnen einer Flüssigkeit, die eine Vorrichtung zum Dosieren nach einem der Ansprüche 7 bis 10 beinhaltet, dadurch gekennzeichnet, daß dasselbe Behältnis (6, 61) zur Aufnahme von sowohl der zu verdünnenden Flüssigkeit als auch des flüssigen Verdünnungsmittels dient.

13. Vorrichtung zum Verdünnen nach Anspruch 7, dadurch gekennzeichnet, daß das Dosismittel oder der Dosisträger (5) eine Kanüle (51) ist, die den kapillaren Kanal (11) bestimmt, und die zwei Öffnungen (21, 22) aufweist, wobei eine mit dem Behältnis (61) in Verbindung steht und die andere mit der Außenseite oder einem Behälter.

14. Vorrichtung nach Anspruch 12, dadurch gekennzeichnet, daß das Behältnis (61) mit einem Mittel (12), das dieses unter Unterdruck und/oder Überdruck setzen kann, und mit einem Mittel (13) zum Einführen und/oder Entleeren der zu verdünnenden Flüssigkeit in Verbindung steht.

## Claims

1. Process for metering a predetermined quantity of a liquid, characterized in that:
(a) at least one capillary cavity (1, 11, 57) is provided which is capable of containing said liquid, the outwardly emerging orifice (2, 21) of which is used both for introducing and emptying said liquid, and is dimensioned such that the liquid cannot enter or leave said cavity spontaneously in particular with cross section less than that of the capillary cavity;
(b) a bath (3, 31) of said liquid is provided;
(c) said cavity (1, 11, 57), initially free of any liquid, is evacuated at least partially in order to bring its residual internal pressure to a value which is less than that prevailing above the liquid bath (3, 31) during step (d), especially atmospheric pressure;
(d) the interior of the capillary cavity (1, 11, 57), which is at least partially evacuated, is connected via the orifice (2, 21) to the liquid bath (3, 31), which is, in particular, under atmospheric pressure, by means of which said cavity is filled;
(e) the filled capillary cavity (1, 11, 57) is separated from the rest of the liquid bath (3, 31);
(f) the capillary cavity (1, 11, 57) which is filled and separated from the liquid bath (3, 31) is emptied.

2. Process according to Claim 1, characterized in that step (c) is carried out by arranging said cavity (1, 11, 57) in the liquid bath (3, 31) and by applying above the latter a pressure less than that prevailing above the liquid bath (3, 31) during step (d).

3. Process according to Claim 1, characterized in that step (f) is carried out according to any one of the modes defined hereinbelow, namely:
3.1) application of a centrifugal force passing through the introduction orifice (2);
3.2) application, outside the cavity (1, 11), of an external pressure less than its internal pressure;
3.3) application, inside the cavity (1, 11), of a pressure greater than its internal pressure;
3.4) the liquid volume filling the cavity (1, 11) is pushed out of the latter.

4. Process according to Claim 3, according to which step (f) is carried out by mode 3.2, characterized in that the cavity (1, 11, 57) is arranged in another liquid bath (4) and a pressure less than the internal pressure of the cavity (1, 11, 57) is applied above said bath.

5. Method of controlled dilution of a liquid, implementing a metering process according to any one of Claims 1 to 4, characterized in that, on the one hand, the liquid (1, 11, 57) to be diluted constitutes the liquid bath according to steps (a) to (e) and, on the other hand, according to step (f), the filled cavity (31) is emptied into a metered quantity of a liquid diluent (4).

6. Method of dilution according to Claims 1, 4 and 5, characterized in that the metered quantity of the liquid diluent (4) constitutes said other liquid bath according to step (f) of the metering process.

7. - Device for metering a predetermined quantity of a liquid, characterized in that it comprises:
(A) a metering means or support (5, 51), used for sampling then releasing the liquid, comprising a capillary cavity (1, 11, 57) capable of containing said liquid, the outwardly emerging orifice (2, 21) of which is used both for introducing and emptying said liquid, dimensioned such that the liquid cannot enter or leave said cavity spontaneously, especially with cross section less than that of the capillary cavity;
(B) a container (6, 61) capable of containing a bath (3, 31) of said liquid, the interior of which can communicate with the interior of the cavity (1, 11, 57) of the metering means, via the orifice (2, 21) of the cavity;
(C) a means (8,12) for evacuating the cavity (1, 11, 57) of the metering means which is initially free of any liquid, via its orifice (2, 21);
(D) a means for separating the filled cavity of the metering means from the rest of the liquid bath (3, 31);
(E) a means for emptying the cavity which is filled with liquid but separated from the rest of the liquid.

8. Metering device according to Claim 7, characterized in that the evacuation means consists of a means (12) for depressurizing the gaseous atmosphere above the container.

9. Device according to Claim 7, characterized in that the means for separating the filled cavity from the rest of the liquid bath is either a means for pumping the latter or a means for suction of the latter through a calibrated orifice of the cavity.

10. Device according to Claim 7, characterized in that the means for emptying the cavity filled with liquid is a means for applying a pressure less than its internal pressure to said cavity.

11. Metering device according to any one of Claims 7 to 11, characterized in that the introduction/emptying orifice (2, 21) has a transverse dimension, such as diameter, which is less than the mean transverse dimension of the capillary cavity, along its length.

12. Device for controlled dilution of a liquid, incorporating a metering device according to any one of Claims 7 to 10, characterized in that the same container (6, 61) is used for receiving both the liquid to be diluted and the liquid diluent.

13. Dilution device according to Claim 7, characterized in that the metering means or support (5) is a needle (51) which defines the capillary conduit (11) and includes two orifices (21, 22), one communicating with the container (61) and the other communicating with the outside or a vessel.

14. Device according to Claim 12, characterized in that the container (61) is in communication with a depressurization and/or pressurization means (12), and a means (13) for introducing and/or removing the liquid to be diluted.
